(19) Europäisches Patentamt
European Patent Office
Office européen des brevets

(11) Veröffentlichungsnummer: **0 050 196**
**B1**

# (12) EUROPÄISCHE PATENTSCHRIFT

(45) Veröffentlichungstag der Patentschrift: **23.11.83**

(51) Int. Cl.³: **C 07 C 103/52,** C 07 C 102/00

(21) Anmeldenummer: **81106217.3**

(22) Anmeldetag: **08.08.81**

(54) **Verfahren zur Herstellung von Peptiden.**

(30) Priorität: **16.10.80 DE 3039053**

(43) Veröffentlichungstag der Anmeldung: **28.04.82 Patentblatt 82/17**

(45) Bekanntmachung des Hinweises auf die Patenterteilung: **23.11.83 Patentblatt 83/47**

(84) Benannte Vertragsstaaten: **BE CH DE FR GB LI NL**

(73) Patentinhaber: **Degussa Aktiengesellschaft, Weissfrauenstrasse 9, D-6000 Frankfurt am Main 1 (DE)**

(72) Erfinder: **Kleemann, Axel, Dr., Dipl.-Chem., Greifenhagenstrasse 9, D-6450 Hanau 9 (DE)**
Erfinder: **Martens, Jürgen Dr., Dipl.-Chem., Hochstrasse 5, D-8755 Alzenau (DE)**
Erfinder: **Samson, Marc, Dr., Grünaustrasse 1, D-6450 Hanau 9 (DE)**

(56) Entgegenhaltungen:
**Chemical Abstracts Band 93, Nr. 22, Dezember 1980 Columbus, Ohio, USA MEYER et al. "Stereoselective synthesis of dipeptides by asymmetric reduction of dehydropeptides catalyzed by chiral rhodium complexes" Abstract Nr. 239917m Seite 906**
**Chemical Abstracts Band 93, Nr. 19, 10. November 1980 Columbus, Ohio, USA OJIMA et al. "Asymmetric synthesis of dipeptides by means of homogeneous hydrogenation catalyzed by chiral rhodium complexes" Seiten 708 Abstract Nr. 186768e**
**JOURNAL OF THE AMERICAN CHEMICAL SOCIETY, Band 100, Nr. 17, August 1978 Gaston FRYZUK et al. "Asymmetric Synthesis. An Asymmetric Homogenous Hydrogenation Catalyst which Breeds its Own Chirality" Seiten 5491 bis 5494**
**Chem. Eng. News, 01.09.1980, Seiten 26, 27**

(56) Entgegenhaltungen: (Fortsetzung)
**Tetrahedron 36, 1980, 815-825**
**Chemistry Letters 1980, 481-482**

EP 0 050 196 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Peptiden

Gegenstand der Erfindung ist ein Verfahren zur Herstellung von Peptiden durch asymmetrische Hydrierung von Dehydropeptiden in Gegenwart von chiralen Rhodium-Komplexen.

Es ist bereits bekannt, Peptide durch asymmetrische Hydrierung von Dehydropeptiden der allgemeinen Formel

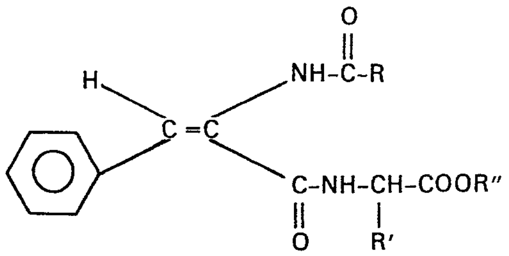

(II)

in der R einen Methyl- oder Phenylrest, R′ einen Alkyl- oder Phenylrest und R″ Wasserstoff oder einen Alkylrest bedeuten, in Gegenwart von chiralen Rhodium-Komplexen herzustellen (Tetrahedron Letters, Vol. 21, Seiten 1239 bis 1242 [1980]; Chemistry Letters 1980, Seiten 481 bis 482). In allen eingesetzten Dehydropeptiden steht die prochirale olefinische Doppelbindung in dem mit einer Aminogruppe terminierten Molekülteil.

Das erfindungsgemässe Verfahren ist nun dadurch gekennzeichnet, dass man zur Hydrierung Dehydropeptide der allgemeinen Formel

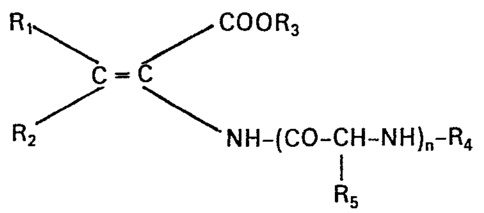

(I)

einsetzt, in der n eine ganze Zahl von 1 bis 4 ist, $R_1$ und $R_2$ gleich oder verschieden sind und Wasserstoff, einen geradkettigen oder verzweigten Alkylrest mit 1 bis 10 Kohlenstoffatomen, einen unsubstituierten oder in 3-, 4-, oder in 3- und 4-Stellung durch eine Hydroxyl-, Alkoxy- oder Acyloxygruppe substituierten Phenylrest oder einen unsubstituierten oder in 6-Stellung durch Fluor, Chlor oder eine Methylgruppe substituierten Indolylrest, $R_3$ Wasserstoff, ein Alkalimetall oder einen niederen Alkylrest mit 1 bis 4 Kohlenstoffatomen, $R_4$ Wasserstoff, einen Acetylrest oder einen Chloracetylrest und $R_5$ Wasserstoff oder einen geradkettigen oder verzweigten Alkylrest mit 1 bis 4 Kohlenstoffatomen bedeuten mit der Massgabe, dass für n = 2, 3 oder 4 die einzelnen Reste $R_5$ gleich oder verschieden sind.

Die beim erfindungsgemässen Verfahren zur Hydrierung einzusetzenden Dehydropeptide können bereits ein oder mehrere asymmetrische Kohlenstoffatome enthalten und in diesem Fall sowohl in Form des Racemats als auch in Form der optisch aktiven Enantiomeren angewandt werden. In jedem Fall wird bei der asymmetrischen Hydrierung ein neues Asymmetriezentrum induziert, und zwar immer in dem mit einer Carboxylgruppe terminierten Molekülteil.

Aus J.A.C.S. 100, 5491 bis 5494, ist zwar auch bereits die asymmetrische Hydrierung verschiedener 2-Acylamino-acrylsäuren bekannt. Dabei wird ebenfalls ein neues Asymmetriezentrum induziert, das in einem mit einer Carboxylgruppe terminierten Molekülteil liegt. Die Dehydropeptide der allgemeinen Formel (I) unterscheiden sich von diesen 2-Acylamino-acrylsäuren wesentlich dadurch, dass sie nicht nur eine, sondern mindestens zwei Amid-Funktionen enthalten, die zudem im Gegensatz zu den Dehydropeptiden der allgemeinen Formel (II) nicht im wesentlichen symmetrisch zu der prochiralen C=C-Doppelbindung angeordnet sind, sondern in zunehmendem Abstand von dieser stehen. Da bekannt ist, dass Amid-Funktionen im Substrat bei der asymmetrischen Hydrierung von prochiralen C=C-Doppelbindungen eine entscheidende Bedeutung zukommt, war es auch im Hinblick auf den insgesamt bekannten Stand der Technik nicht vorhersehbar, dass sich die Dehydropeptide der allgemeinen Formel (I) trotz ihres im Vergleich mit den bekannten Substraten völlig anderen Aufbaus komplikationslos in Gegenwart eines chiralen Katalysators asymmetrisch unter bevorzugter Bildung eines der beiden möglichen Enantiomeren hydrieren lassen.

Die Dehydropeptide werden mit einer freien Carboxylgruppe, als Alkalimetallsalz oder als Ester mit einem Akanol mit 1 bis 4, vorzugsweise 1 bis 2, Kohlenstoffatomen zur Hydrierung eingesetzt. Mit besonders gutem Erfolg lassen sich beim erfindungsgemässen Verfahren solche Dehydropeptide hydrieren, für die in der obigen allgemeinen Formel (I) n die Bedeutung 1 oder 2 hat. Als $R_4$ werden Acetyl- oder Chloracetylreste bevorzugt.

Beispiele für nach dem erfindungsgemässen Verfahren hydrierbare Dehydropeptide sind Glycyl-dehydroleucin, N-Acetyl-glycyl-dehydroleucin, N-Chloracetyl-glycyl-dehydroleucin, Glycyl-dehydrophenylalanin, N-Acetyl-glycyl-dehydrophenylalanin, N-Chloracetyl-glycyl-dehydrophenylalanin, Glycyl-glycyl-dehydroalanin, N-Acetyl-glycyl-dehydroalanin, N-Chloracetyl-glycyl-dehydroalanin, N-Acetyl-glycyl-glycyl-dehydroleucin, N-Acetyl-glycyl-glycyl-dehydrophenylalanin, N-Chlor-acetyl-alanyl-dehydroalanin, N-Chloracetyl-glycyl-dehydrotryptophan, N-Acetyl-glycyl-dehydrotryptophan, N-Acetyl-glycyl-dehydroisoleucin, N-Acetyl-glycyl-dehydrotyrosin, N-Acetyl-glycyl-dehydroglutaminsäure, N-Chloracetyl-glycyl-dehydrotyrosin, Glycyl-dehydrotryptophan, Glycyl-dehydroglutaminsäure oder Glycyldehydrotyrosin.

Die einzusetzenden Dehydropeptide können beispielsweise so hergestellt werden, dass man im einfachsten Falle der Glycyl-dehydroaminosäure, für die in Formel (I) n = 1 und $R_5$ = H ist,

eine Aminosäure mit Dichloracetylchlorid acyliert, die gebildete N-Dichloracetyl-aminosäure mittels eines Carbonsäureanhydrids, vorzugsweise Essigsäureanhydrid, in das entsprechende 4-Alkyliden- bzw. 4-Arylalkyliden-2-chlormethyl-oxazolon umwandelt, dieses zu der entsprechenden N-Chloracetyl-dehydroaminosäure hydrolysiert und diese letztere mit Ammoniak zu der entsprechenden Glycyl-dehydroaminosäure umsetzt. Diese letztere kann dann wieder im einfachsten Falle mit Chloracetyl-chlorid zu der entsprechenden N-Chloracetyl-glycyl-dehydroaminosäure umgesetzt und diese mit Ammoniak in die entsprechende Glycyl-glycyl-dehydroaminosäure umgewandelt werden, für die in Formel (I) $n = 2$ und $R_5 = H$ ist. Die ein- oder zweimalige Wiederholung der beiden letztgenannten Reaktionsschritte führt dann schliesslich zu Dehydropeptiden, für die in Formel (I) $n = 3$ oder 4 und $R_5 = H$ ist. Geht man anstatt von einer Aminosäure und Dichloracetylchlorid von einer Aminosäure und einem α,α-Dichloracylchlorid mit 3 bis 6 Kohlenstoffatomen aus und/oder verwendet man anstelle von Chloracetylchlorid ein höheres α-Chloracylchlorid mit 3 bis 6 Kohlenstoffatomen, so lassen sich auf die gleiche Weise entsprechende Dehydropeptide herstellen, für die in Formel (I) $R_5$ einen Alkylrest mit 1 bis 4 Kohlenstoffatomen bedeutet. Die jeweils endständige Aminogruppe des Dehydropeptids kann dann schliesslich mit Essigsäureanhydrid, Acetylchlorid oder Chloracetylchlorid acyliert und/oder die freie Carboxylgruppe in ein Alkalimetallsalz oder in eine Estergruppe umgewandelt werden.

Die asymmetrische Hydrierung der Dehydropeptide erfolgt in Gegenwart von löslichen Koordinationskomplexen aus einer Rhodium-(I)-Verbindung und einem chiralen tertiären Phosphin. Derartige Komplexe können in situ hergestellt werden durch die Umsetzung eines chiralen tertiären Phosphins mit einem Komplex der allgemeinen Formel

$$[Rh(en)_2X]_2,$$

in der $(en)_2$ zwei Moleküle eines Monoolefins oder ein Molekül eines Diolefins und X Chlor, Brom oder Jod bedeuten, oder mit einem Komplex der allgemeinen Formel

$$[Rh(en)_2Y]$$

in der $(en)_2$ wieder die bereits angegebene Bedeutung hat und Y einen Acetonylacetonat- oder einen Carboxylatrest bedeutet.

Als Katalysatoren für die asymmetrische Hydrierung sind schliesslich auch kationische Komplexe der allgemeinen Formel

$$[Rh(en)_2A]^+Z^-$$

verwendbar, in der $(en)_2$ wieder die bereits angegebene Bedeutung hat, A ein chirales, tertiäres Phosphin und $Z^-$ ein Tetrafluoroborat-, Tetraphenylborat-, Hexafluorophosphat- oder Perchlorat-Anion bedeutet.

Der Begriff «chirales tertiäres Phosphin» bezeichnet entweder ein Phosphin, dessen Phosphoratom drei verschiedene, gegebenenfalls substituierte, Kohlenwasserstoffreste trägt, oder ein Phosphin, welches drei, gegebenenfalls substituierte, Kohlenwasserstoffreste trägt, von denen mindestens einer chiral ist. Beispiele für solche chiralen tertiären Phosphine sind

(+)-2,3-O-Isopropyliden-2,3-dihydroxy-1,4-bis-(diphenylphosphino)-butan,
(−)-1,2-Bis-(O-anisylphenylphosphino)-äthan,
(−)-(2R,3R)-Bis-(diphenylphosphino)-bicyclo-[2.2.1]-hept-5-en,
(S)-(α)-[(R)-1′,2′-Bis-(diphenylphosphino)-ferrocenyl]-äthyldimethylamin,
(S,S)-N,N′-Bis-(S)-α-methylbenzyl-N,N′-bis-(diphenyl-phosphino)äthan,
(2S,4S)-N-Butoxycarbonyl-4-diphenylphosphino-2-diphenylphosphino-methylpyrrolidin,
(R)-1,2-Bis-(diphenylphosphinomethyl)-cyclobutan,
(R)-(+)-Methylpropyl-o-dimethylaminophenylphosphin,
(+)- und (−)-Neomenthyl-diphenylphosphin,
(S)-(+)- und (S)-(−)-1,2-Bis-(diphenylphosphino)-1-phenyläthan,
(2R,4R)-Bis-(diphenylphosphinomethyl)-dioxolan,
(1R,2R)-Bis-(N-diphenylphosphinomethylamino)-cyclohexan,
(1R,2R)-Bis-(N-diphenylphosphino-amino)-cyclohexan,
(S)-(−)-2,2′-Bis-(diphenylphosphinomethyl)-1,1′-binaphthyl,
(S,S)-2,3-Bis-(diphenylphosphino)-butan,
(R)-1,2-Bis-(diphenylphosphino)-propan,
6-(S)-Cyano-5-(R)-diphenylphosphino-3R,4S-O-iso-propyliden-2-oxabicyclo-[3.2.0]-heptan.

Die asymmetrische Hydrierung wird in den bei Hydrierungen gebräuchlichen Lösungsmitteln, wie Alkoholen und Äthern, oder deren Gemischen mit aliphatischen oder aromatischen Kohlenwasserstoffen, oder in Wasser, durchgeführt. Auch Gemische aus Alkoholen und Wasser können verwendet werden.

Die Konzentration an dem zu hydrierenden Dehydropeptid kann von einer 0,001 molaren bis zu einer an dem betreffenden Dehydropeptid übersättigten Lösung reichen. Der Wasserstoffdruck kann zwischen Normaldruck und etwa 100 bar, vorzugsweise zwischen Normaldruck und 25 bar, liegen, die Reaktionstemperatur zwischen −20° und +50°C. Vorzugsweise wird die Hydrierung bei Raumtemperatur vorgenommen.

Die chiralen Rhodium-Komplexe werden zweckmässig in solcher Menge eingesetzt, dass das Molverhältnis von Dehydropeptid zu Katalysator im Bereich zwischen 1:1 und 50 000:1, vorzugsweise zwischen 100:1 und 5000:1, liegt.

Die nach dem erfindungsgemässen Verfahren hergestellten Peptide dienen als pharmazeutische Wirkstoffe oder als Zwischenprodukte beim Aufbau höherer Peptide.

Das erfindungsgemässe Verfahren wird durch die nachfolgenden Beispiele näher erläutert, ohne dass dadurch der Umfang der Erfindung beschränkt werden soll. Die in den Beispielen angegebene optische Ausbeute ist wie folgt definiert:

$$\text{Optische Ausbeute (\%)} = \frac{\text{Gemessene optische Aktivität des erhaltenen Gemisches}}{\text{optische Aktivität des reinen Enantiomorphen}} \times 100$$

Beispiel 1

3,7 g N-Acetyl-glycyl-dehydroleucin wurden in 50 ml luftfreiem Äthanol aufgeschlämmt. Dieser Aufschlämmung wurden 50 ml einer äthanolischen Lösung zugesetzt, die pro ml 5,6 mg [Rh(COD)-(+)-DIOP] $BF_4$ enthielt [(+)-DIOP steht für (+)-2,3-O-Isopropyliden-2,3-dihydroxy-1,4-bis-(diphenylphosphino)-butan und (COD) für Cyclooctadien].

Das Gemisch wurde bei 25°C 8 Stunden lang unter Wasserstoff geschüttelt, wobei der Wasserstoffdruck von anfänglich 10 bar auf 6,9 bar sank. Anschliessend wurde das Reaktionsgefäss belüftet und das Lösungsmittel unter vermindertem Druck entfernt. Als Rückstand verblieb in Form weisser Kristalle in nahezu quantitativer Ausbeute rohes N-Acetyl-glycyl-leucin mit einer spezifischen Drehung $[\alpha]_D^{25}$ von −6,7° (c = 1;$H_2O$). Mit einem maximalen Drehwert $[\alpha]_D^{25}$ von −25,3° (J. biol. Chem. 132, 167 [1940]) entspricht dies einer optischen Ausbeute von 27% mit (S)-Konfiguration.

Beispiel 2

2,35 g N-Acetyl-glycyl-dehydroleucin wurden in 50 ml luftfreiem Äthanol gelöst und mit 5,0 ml einer äthanolischen Lösung versetzt, die pro ml 4 mg [Rh(COD)-(−)-BPPM]$BF_4$ enthielt [(−)-BPPM steht für (2S,4S)-N-Butoxycarbonyl-4-diphenylphosphino-2-diphenylphosphino-methyl-pyrrolidin].

Die Lösung wurde bei 25°C 8 Stunden lang unter Wasserstoff geschüttelt, wobei der Wasserstoffdruck von anfänglich 12,5 bar auf 8,7 bar sank. Anschliessend wurde das Reaktionsgefäss belüftet und das Lösungsmittel unter vermindertem Druck entfernt. Als Rückstand verblieb in Form einer weissen Kristallmasse in nahezu quantitativer Ausbeute rohes N-Acetyl-glycyl-leucin mit einer spezifischen Drehung $[\alpha]_D^{25}$ von +14,7° (c = 1;$H_2O$). Dies entspricht einer optischen Ausbeute von 58% mit (R)-Konfiguration.

Beispiel 3

2,8 g N-Acetyl-glycyl-dehydroleucin wurden in 50 ml luftfreiem Äthanol gelöst und mit 5,0 ml einer äthanolischen Lösung versetzt, die pro ml 3,5 mg [Rh(COD)-(R,R-2)]$BF_4$ enthielt [(R,R-2) steht für (1R,2R)-Bis-(diphenylphosphinoamino)-cyclohexan].

Die Lösung wurde bei 25°C 8 Stunden lang unter Wasserstoff geschüttelt, wobei der Wasserstoffdruck von anfänglich 11 bar auf 7,3 bar sank. Anschliessend wurde das Reaktionsgefäss belüftet und das Lösungsmittel unter vermindertem Druck entfernt. Als Rückstand verblieb in Form einer weissen Kristallmasse in praktisch quantitativer Ausbeute rohes N-Acetyl-glycyl-leucin mit einer spezifischen Drehung $[\alpha]_D^{25}$ von +9,5° (c = 1;$H_2O$). Dies entspricht einer optischen Ausbeute von 37,5% mit (R)-Konfiguration.

Beispiele 4 bis 6

Analog wie in den Beispielen 1 bis 3 wurden jeweils 1,4 g N-Chloracetyl-glycyl-dehydroleucin hydriert. Die Reaktionsbedingungen und Ergebnisse sind in Tabelle I zusammengefasst.

Tabelle 1

| Substrat | Produkt | Katalysator | $H_2$-Druck (bar) | $\alpha_D^{25}$ |
|---|---|---|---|---|
| N-Chloracetyl-glycyl-dehydroleucin | N-Chloracetyl-glycyl-leucin | A | 10 | − 6,0° |
| | | B | 12 | +13,8° |
| | | C | 20 | +10,4° |

A : [RH(COD)-(+)-DIOP]$BF_4$
B : [RH(COD)-(−)-BPPM]$BF_4$
C : [Rh(COD)-(R,R-2)]$BF_4$
$\alpha_D^{25}$ : gemessen in EtOH (c = 1)

Beispiele 7 bis 9

Jeweils 4,0 g entgastes N-Acetyl-glycyl-dehydrophenyl-alanin wurden in 75 ml Äthanol aufgeschlämmt und mit 5,0 ml einer äthanolischen Lösung versetzt, die pro ml 4 mg Katalysator enthielt. Die Hydrierungen wurden bei 25°C vorgenommen. Nach dem Ende der Reaktionszeit wurde jeweils das Reaktionsgefäss belüftet und das Lösungsmittel unter vermindertem Druck entfernt. In allen Fällen hinterblieb in Form einer

weissen Kristallmasse in praktisch quantitativer Ausbeute rohes N-Acetyl-glycyl-phenyl-alanin.

Die Reaktionsbedingungen und Ergebnisse sind in Tabelle II zusammengefasst.

Tabelle 2

| Katalysator | $H_2$-Druck (bar) | Reaktions-zeit (h) | $\alpha_D^{25}$ | Optische Ausbeute | Konfigura-tion |
|---|---|---|---|---|---|
| A | 20 | 17 | +19,5° | 44,3% | (S) |
| B | 15 | 14 | −27,7° | 63% | (R) |
| C | 25 | 24 | −17,3° | 39,3% | (R) |

A : $[Rh(COD)\text{-}(+)\text{-}DIOP]BF_4$
B : $[RH(COD)\text{-}(-)\text{-}BPPM]BF_4$
C : $[Rh(COD)\text{-}(R,R\text{-}2)]BF_4$
$\alpha_D^{25}$ : gemessen in EtOH (c = 2) [Maximaler Drehwert: +44° (c = 2, EtOH) J. Chem. Soc. (C), 1967, 595-601].

Beispiele 10 bis 12

Analog wie in den Beispielen 7 bis 9 wurden jeweils 4,3 g N-Chloracetyl-glycyl-dehydrophenylalanin hydriert. Die Reaktionsbedingungen und Ergebnisse sind in der nachstehenden Tabelle III zusammengefasst.

Tabelle 3

| Katalysator | Druck (bar) | Reaktionszeit (h) | $\alpha_D^{25}$ | wahrscheinliche Konfiguration |
|---|---|---|---|---|
| A | 15 | 18 | +18,5° | (S) |
| B | 20 | 10 | −23,3° | (R) |
| C | 15 | 24 | −14,2° | (R) |

A : $[Rh(COD)\text{-}(+)\text{-}DIOP]BF_4$
B : $[RH(COD)\text{-}(-)\text{-}BPPM]BF_4$
C : $[RH(COD)\text{-}(R,R\text{-}2)]BF_4$
$\alpha_D^{25}$ : gemessen in EtOH (c = l)

**Patentanspruch**

Verfahren zur Herstellung von Peptiden durch asymmetrische Hydrierung von Dehydropeptiden in Gegenwart von chiralen Rhodium-Komplexen, dadurch gekennzeichnet, dass man zur Hydrierung Dehydropeptide der allgemeinen Formel

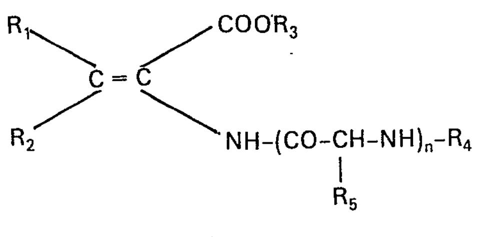

(I)

einsetzt, in der n eine ganze Zahl von 1 bis 4 ist, $R_1$ und $R_2$ gleich oder verschieden sind und Wasserstoff, einen geradkettigen oder verzweigten Alkylrest mit 1 bis 10 Kohlenstoffatomen, einen unsubstituierten oder in 3-, 4- oder in 3- und 4-Stellung durch einen Hydroxyl-, Alkoxy- oder Acyloxygruppe substituierten Phenylrest oder einen unsubstituierten oder in 6-Stellung durch Fluor, Chlor oder eine Methylgruppe substituierten Indolylrest, $R_3$ Wasserstoff, ein Alkalimetall oder einen niederen Alkylrest mit 1 bis 4 Kohlenstoffatomen, $R_4$ Wasserstoff, einen Acetylrest oder einen Chloracetylrest und $R_5$ Wasserstoff oder einen geradkettigen oder verzweigten Alkylrest mit 1 bis 4 Kohlenstoffatomen bedeuten mit der Massgabe, dass für n = 2, 3 oder 4 die einzelnen Reste $R_5$ gleich oder verschieden sind.

**Revendication**

Procédé pour la préparation de peptides par hydrogénation asymétrique de déshydropeptides en présence de complexes de rhodium chiraux, caractérisé en ce qu'on utilise pour l'hydrogénation des déshydropeptides de formule générale

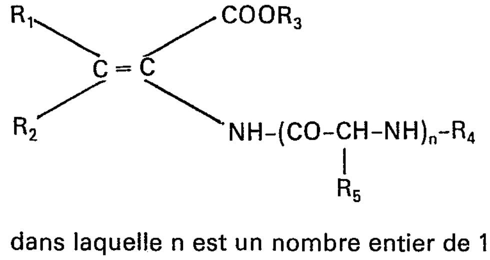

(I)

dans laquelle n est un nombre entier de 1 à 4, $R_1$ et $R_2$ sont identiques ou différents et représentent l'hydrogène, un reste alkyle linéaire ou ramifié comportant 1 à 10 atomes de carbone, un reste phényle non substitué ou substitué en position 3, 4 ou 3 et 4 par un groupe hydroxyle, alkoxy ou acyloxy ou un reste indolyle non substitué ou

substitué en position 6 par le fluor, le chlore ou un groupe méthyle, $R_3$ représente l'hydrogène, un métal alcalin ou un reste alkyle inférieur comportant 1 à 4 atomes de carbone, $R_4$ représente l'hydrogène, un reste acétyle ou chloro-acétyle et $R_5$ représente l'hydrogène ou un reste alkyle linéaire ou ramifié comportant 1 à 4 atomes de carbone, les différents restes $R_5$ étant identiques ou différents lorsque n = 2, 3 ou 4.

**«A process for the production of peptides»**
**Claim**

A process for the production of peptides by asymmetric hydrogenation of dehydropeptides in the presence of chiral rhodium complexes, characterised in that for the hydrogenation, dehydropeptides corresponding to the general formula are used:

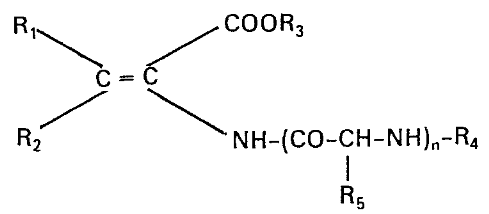

(I)

wherein n represents an integer from 1 to 4, $R_1$ and $R_2$ are the same or different, and represent hydrogen, a straight- or branched-chain alkyl radical having from 1 to 10 carbon atoms, an unsubstituted phenyl radical or a phenyl radical substituted in the 3-, 4- or in the 3- and 4-position by a hydroxyl, alkoxy or acyloxy group, or an unsubstituted indolyl radical or an indolyl radical substituted in the 6-position by fluorine, chlorine or a methyl group, $R_3$ represents hydrogen, an alkali metal or a lower alkyl radical having from 1 to 4 carbon atoms, $R_4$ represents hydrogen, an acetyl radical or a chloroacetyl radical, and $R_5$ represents hydrogen or a straight- or branched-chain alkyl radical having from 1 to 4 carbon atoms, provided that when n = 2, 3 or 4, the individual radicals $R_5$ are the same or different.